# EUROPEAN PATENT SPECIFICATION

(11) **EP 2 297 333 B1**
(45) Date of publication and mention of the grant of the patent: **28.01.2015**
(21) Application number: 09755288.9
(22) Date of filing: 01.06.2009
(51) Int. Cl.: C12Q 1/24, C12M 1/34, G01N 33/52, C12N 15/10

(54) **METHOD FOR SPATIAL SEPARATION AND SCREENING OF CELLS**
VERFAHREN ZUR RÄUMLICHEN TRENNUNG UND ZUM SCREENING VON ZELLEN
PROCÉDÉ POUR UNE SÉPARATION SPATIALE ET UN CRIBLAGE DE CELLULES

(30) Priority: 30.05.2008 US 57371 P
(43) Date of publication of application: 23.03.2011
(73) Proprietor: Massachusetts Institute of Technology, Cambridge, MA 02139-4307 (US); Whitehead Institute For Biomedical Research, Cambridge, MA 02142 (US)
(72) Inventor: LOVE, J. Christopher, Somerville, MA 02143 (US); LOVE, Kerry, Somerville, MA 02143 (US)
(74) Representative: Tostmann, Holger Carl
(86) International application number: PCT/US2009/003354
(87) International publication number: WO 2009/145925

(56) References cited:
- WO-A2-2007/035633
- US-B1- 6 576 478
- FUKUDA ET AL: "Development of high-throughput screening system by single-cell reaction using microchamber array chip", JOURNAL OF BIOSCIENCE AND BIOENGINEERING, ELSEVIER, AMSTERDAM, NL, vol. 104, no. 3, 1 September 2007 (2007-09-01), pages 241-243, XP022314956, ISSN: 1389-1723, DOI: DOI:10.1263/JBB.104.241
- DAVIS J M ET AL: "A simple, single-step technique for selecting and cloning hybridomas for the production of monoclonal antibodies", JOURNAL OF IMMUNOLOGICAL METHODS, ELSEVIER SCIENCE PUBLISHERS B.V.,AMSTERDAM, NL, vol. 50, no. 2, 29 April 1982 (1982-04-29) , pages 161-171, XP023676288, ISSN: 0022-1759, DOI: DOI:10.1016/0022-1759(82)90222-8 [retrieved on 1982-04-29]
- J. CHRISTOPHER LOVE ET AL.: 'A microengraving method for rapid selection of single cells producing antigen-specific antibodies.' NATURE BIOTECHNOLOGY vol. 24, no. 6, June 2006, pages 703 - 707, XP002546224
- GARVIN J. WILLIAMS ET AL.: 'A high-throughput fluorescence-based glycosytransferase screen and its application in directed evolution.' NATURE PROTOCOLS. vol. 3, no. 3, 14 February 2008, pages 357 - 362, XP008099796
- GAVIN J. WILLIAMS ET AL.: 'Expanding the promiscuity of a natural-product glycosyltransferase by directed evolution.' NATURE CHEMICAL BIOLOGY. vol. 3, no. 10, October 2007, pages 657 - 662, XP002508684
- BRIGITTE POHN ET AL.: 'Micro-colony based high throughput platform for enzyme library screening.' JOURNAL OF BIOTECHNOLOGY. vol. 129, no. 1, March 2007, pages 162 - 170, XP008138572

## Description

### FIELD OF THE INVENTION

The invention is based on isolating particular members from a library of variant cells in individual microreactors, wherein the phenotype of the biomolecule encoded by the cell is evaluated on the basis of multiple parameters, including substrate specificity and kinetic efficiency.

### BACKGROUND OF THE INVENTION

Enzymes are increasingly being used as catalysts in industry, agriculture, medicine and scientific research. Due to their substrate specificity, chemical selectivity and environmental compatibility, enzymes offer advantages for such applications as the synthesis of chirally pure pharmaceuticals, textile processing, food processing, medical diagnostics and therapy, biotransformation and bioremediation. Enzymes are proving to be superior to traditional chemical processes for modifying high molecular weight polymers.

Evaluation of libraries of genetic variants of biomolecules, such as enzymes, to identify specific members in the library with desired properties requires both characterizing the phenotype of the biomolecule produced and correlating the biomolecule to the genotype of the member of the library encoding it. In this way, desired variants are selected and further evaluated. Directed evolution has proven particularly successful in cases where enzyme function is directly linked to cell survival, *i.e.,* restoration of an essential activity that has been deleted from an otherwise wild-type cell. However, evolution of enzymes that do not themselves provide a selectable phenotype, as in the case of glycosyltransferases (GTases) and other transferases, is much more difficult. While selection strategies do exist to evolve enzymes of this sort, including chemical complementation, phage display and bacterial cell surface display, current methods do not provide a facile or generalized strategy for engineering diverse enzymes. As the demand for new biomolecules grows, there is a pressing need for new strategies for engineering enzymes with improved activity and novel catalytic function.

An article by Love et al. [Nature Biotechnology, 24(6). 2006, pages 703-707] and WO 2007/035633, both also cited further below in the present patent, demonstrate the ability to detect secreted products from individual yeast cells. An article by Fukuda et al (J. Bioscience Bioengineer, 104(3), 2007, pages 241-243) shows proof-of-principle for high throughput screening systems for the rapid screening of single cell combinatorily mutated β-glucosidase. An article by G. Williams and J.S. Thorson (Nature Protocols, Vol. 3,2008, pages 357-361) discloses the application of a high throughput fluorescence based screen for altering the proficiency of secondary metabolite glycosyltransferase.

### SUMMARY OF THE INVENTION

The methods of the invention are based on isolating particular members from a library of variant cells in individual microreactors, wherein the phenotype or activity of the biomolecule encoded by the cell is evaluated on the basis of multiple parameters, including substrate specificity and kinetic efficiency.

In one aspect, the present disclosure relates to methods for screening libraries of secreted products for novel phenotypes, including enzymes with improved catalytic properties or altered substrate specificity using microwells for the special separation of cells producing the enzymes.

In accorance with claim 1, the invention provides for methods of performing enzyme screening in solution phase, comprising depositing a library of cells onto a microdevice, wherein the microdevice contains a plurality of wells that spatially separate the cells in solution. The cells are distributed at on average-one cell per well, and a plurality of cells secrete variants of at least one enzyme in the solution. The secreted enzyme variants are contacted with at least one optical signal substrate. Generally, the optical signal is indicative of a desired biomolecule phenotype or activity; and the phenotype of the biomolecule encoded by the cell is evaluated on the basis of multiple parameters. In some cases, the "optical signal substrate" is a composite of one or more units, e.g., an antibody or other specific ligand or small molecule tag that is directly conjugated to a detectable marker. For example, in a two element reaction (e.g., X + Y catalyzed by a transferase enzyme), a first element, "Y", is captured by an antibody or other ligand that is immobilized on a surface such as a culture plate and the second element, "X", is detected with an optical substrate such as a fluorescently-tagged antibody. The cells that secrete a desired biomolecule variant from the microdevice are then isolated.

Optionally, the phenotype is evaluated by detecting changes over time in one or more optical signals generated by one or more optical signal substrates in the library of cells, wherein such changes indicate desired biomolecule phenotype or activity of the variants of the biomolecule. The invention utilizes various chromogenic, fluorogenic, lumigenic and fluorescence resonance energy transfer (FRET) substrates to measure biological activity. Many donor/acceptor FRET pairs are commercially available. These include, but are not limited to: 5-carboxytetramethylrhodamine (TAMRA)/ QSY-7 (diarylrhodamine derivative); Dansyl / Eosin; Tryptophan / Dansyl; Fluorescein / Texas Red (rhodamine); Naphthalene/Dansyl; Dansyl / octadecylrhodamine (ODR); boron-dipyrromethene (BODIPY) / BODIPY; Terbium / Thodamine; Dansyl / fluorescein isothiocyanate (FITC); Pyrere / Coumarin; 5-(2-iodoacetylaminoethyl)aminonaphthalene-1-sulfonic acid (IAEDANS) / IAFBPE / Cy5; and Europium / Cy5. Preferably, the optical signal is a fluorescence signal. In one aspect, the biomolecule phenotype or activity is monitored in real-time or near-real-time in the microdevice on the basis of changes in the intensities of the fluorescent signal.

The present disclosure provides that the biomolecule is selected from the group consisting of a secreted molecule, a peptide, a polypeptide, an enzyme such as a protease, an oxidoreductase, a transferase, a hydrolase, a hydrogenase, a lyase, an isomerase, a ligase, a polymerase, as well as an antibody, a cytokine, a chemokine a nucleic acid, a metabolite, a small molecule (<1 kDa) and a synthetic molecule. In accordance with claim 1, the biomolecule is an enzyme. For example, the molecular weight of the biomolecule is greater than about 100 Da and less than about 100,000 Da. Alternatively, the molecular weight of the biomolecule is greater than about 600 Da and less than about 30,000 Da; greater than about 800 Da and less than about 10,000 Da; or greater than about 900 Da and less than about 1,000 Da.

In one approach, activity of the enzyme biomolecule is evaluated by detecting the proximity of two or more elements upon which the enzyme or other biomolecule acts. For example, the enzyme brings together the elements (*e.g.,* ligase) or separates the elements (*e.g.,* lyase). As described above, detection is accomplished using FRET pairs or a capture based assay in which a first element is biotinylated (and captured with an avidin-based reagent) and a second element is labeled with a fluorescent tag. An increase or decrease in the association of the elements (substrates) reflects altered binding specificity/activity of the enzyme.

The present disclosure relates to evaluating the phenotype of the biomolecule encoded by the cell on the basis of multiple parameters, wherein the parameters are selected from the group consisting of catalytic rate, specificity of reaction, kinetic efficiency, and substrate binding affinity. In another aspect, rate or substrate tolerance, and pH or temperature tolerance are evaluated. Preferably, the parameters are evaluated in parallel.

The disclosure provides for screening biomolecules secreted by cells. In one aspect, the cells are eukaryotic cells. Preferably, the eukaryotic cells are yeast cells. Alternatively, the cells are prokaryotic cells.

The disclosure also provides for a microdevice that contains wells that spatially separate the cells in solution, *e.g.,* each well contains solely a single cell. Preferably, the wells are between about 10 and about 100 µm in diameter, *e.g.,* 10 µm, 20 µm, 30 µm, 50 µm, or 75 µm in diameter.

In one aspect, the disclosure provides for isolating the cells that secrete a desired biomolecule variant from the microdevice. Preferably, the cells are isolated by micromanipulation with a glass capillary. Optionally, the disclosureprovides for randomly mutagenizing the desired biomolecule for further selection. Suitable techniques for random mutagenesis include error-prone polymerase chain reaction (PCR), codon cassette mutagenesis, deoxyribonucleic acid (DNA) shuffling, staggered extension process (StEP), chemical mutagenesis and the use of mutator strains. Alternatively, the biomolecule is sequenced to identify the biomolecule.

Biomolecules to be interrogated include enzymes. For example, the biomolecule is a mutant glycosyltransferase (GTase), a carbohydrate processing enzyme, a carbohydrate binding protein, a glycosidase, or a lectin affinity protein that binds carbohydrates. Preferably, the GTase is capable of competing with chemical synthesis for the rapid and large scale production of complex carbohydrates. Alternatively, the biomolecules are cytokines, chemokines, antibodies, or other secreted cell metabolites.

In yet another aspect, the disclosure provides for directed evolution of existing GTases to identify more potent catalysts with altered substrate selectivity. More specifically, the invention provides for the identification of mutant GTases capable of competing with chemical synthesis for the rapid and large scale production of glycoconjugates for therapeutic purposes, including carbohydrate-based cancer vaccines and carbohydrate-containing antibiotics.

Other features and advantages of the invention will be apparent from the following description of the preferred embodiments thereof, and from the claims.

### BRIEF DESCRIPTION OF THE DRAWINGS

Figure 1 is a schematic illustration of a method for identifying enzymes with new or improved function. Yeast cells secrete proteins of interest within the microreactors. As every cell is contained within its own well, each well corresponds to a single library member. Following the screening of the invention, the cells are retrieved and used either in further rounds of screening or for identification of the encoded protein.
Figure 2 is a schematic illustrating mucin-type *O*-linked glycans.
Figure 3 is a schematic illustrating substrates for the detection of tobacco etch virus (TEV) protease catalytic activity containing a dipyrromethene boron difluoride (BODIPY) fluorophore (F) and a tetramethylrhodamine (TAMRA) quencher (Q).
Figure 4 is a schematic showing substrates for the detection of ppGalNAcTase-T1 catalytic activity.
Figure 5 is a series of diagrams; (A) is a schematic illustrating an exemplary antitumor vaccine; (B) is a schematic showing an exemplary antiparasitic vaccine; (C) is a schematic illustrating an exemplary antimicrobial vaccine; and (D) is a schematic illustrating an exemplary antimicrobial agent.
Figure 6 is a diagram that demonstrates the structural comparison of the following glycosyltransferases: BTG, MurG, and GtfB.
Figure 7 is a schematic illustration of directed evolution for enzyme engineering and catalyst development.
Figure 8A is a schematic illustration of a method for correlating proteins with the cells that secrete them, in which substrates/products are captured on the contacted glass surface; (B) is a photograph of a device containing wells between 50 and 100 µm in diameter; (C) is a photomicrograph of a protein microarray of secreted products from *Pichia pastoris* cells; and (D) is a photomicrograph of *Pichia pastoris* cells in microwells.
Figure 9 is a photomicrograph of a standard curve for the comparison of protein secretion levels between different cell typres, such as hybridomas, *Pichia pastoris,* and cytokine-secreting peripheral blood mononuclear cells (PBMC).
Figure 10 is a series of photomicrographs demonstrating cell retrieval using a micromanipulator.
Figure 11 is a diagram showing a method for detecting enzyme turnover in microwells via a trypsin cleavage assay.
Figure 12 is a series of photomicrographs demonstrating fluorescent signal intensity after increasing concentrations (0.05 µg/ml, 0.5 µg/ml, and 5 µg/ml) of trypsin were incubated with 10µg/ml FTC casein for 1 hour microwells.
Figure 13 is a series of photomicrographs depicting fluorescent signal intensity after 0.5 µg/ml of trypsin was incubated with 10 µg/ml FTC-casein for 1 and 18 hours in microwells.
Figure 14 is a diagram showing a method for detecting enzyme turnover in microwells via an HRV-3C protease assay.
Figure 15 is a series of photomicrographs showing the results of the HRV-3C protease assay after incubation in 100 µg/ml FRET peptide in 1X reaction buffer containing media (YPD media) for 18 hours at room temperature (RT).

### DETAILED DESCRIPTION OF THE INVENTION

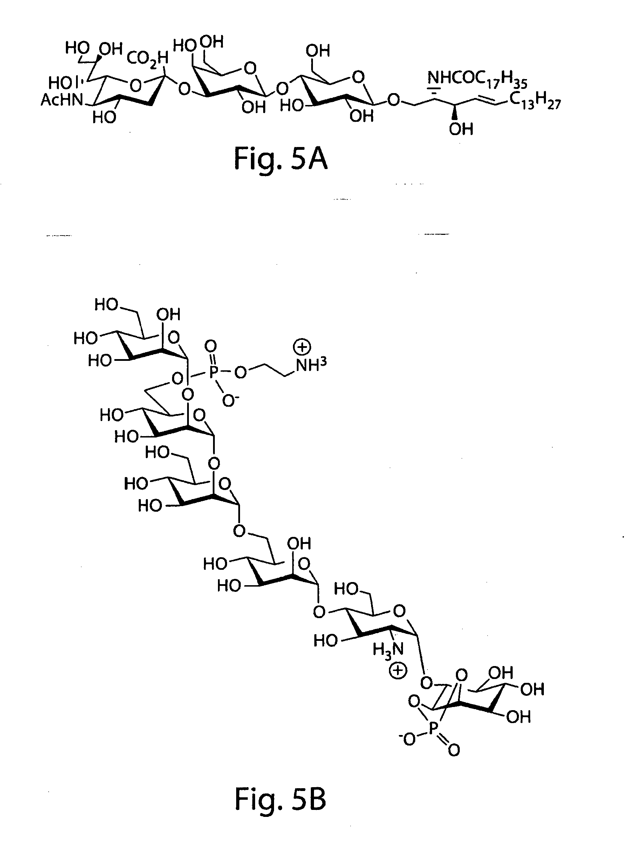

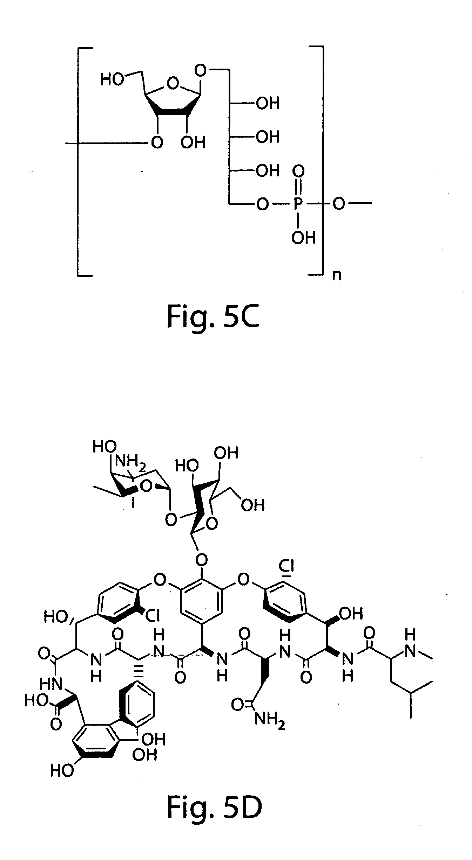

Due to their substrate specificity, chemical selectivity and environmental compatibility, enzymes offer advantages for such applications as the synthesis of chirally pure pharmaceuticals useful in medical diagnostics and therapy. Indeed, such enzymes are utilized in the synthesis of oligosaccharides and glycoconjugates, which have diverse medical applications, including antitumor vaccines (targeting, *e.g.,* GM3, a melanoma-related glycosphingolipid), antiparasitic vaccines (targeting, *e.g.,* malarial glycosylphosphatidylinositol (GPI anchor), antimicrobial vaccines (targeting, *e.g.,* capsular polysaccharide antigen *Haemophilus influenzae* serotype b (HIB)), and other antimicrobial agents. Exemplary antitumor vaccines, antiparasitic vaccines, antimicrobial vaccines, and antimicrobial agents are shown in Figures 5A-5D, respectively. Use of glycosylated biomolecules requires not only intimate knowledge of structural and functional relationships, but also access to defined structures for large scale clinical use.

Although many wild-type enzymes (*i.e.,* those whose amino acid sequences are the same as those found in naturally occurring organisms) can be used without any modification, there are many instances wherein the physical properties of an enzyme or its chemical activity are not compatible with a desired application. Novel physical properties which might be desirable could include, for example, thermal stability, resistance to non-aqueous solvents, salt, metals, inhibitors, proteases, extremes of pH and the like. Reducing the size of the enzyme, abolishing its dependence on cofactors or other proteins, improving its expression in the host strain and other similar changes might also be desirable for a particular application. Improved chemical activities might include, for example, enhanced catalytic rate, substrate affinity and specificity, regioselectivity, enantioselectivity, reduced product inhibition, or an altered pH-activity profile. In addition, it may be desirable to alter the properties of one or more enzymes that function together as part of a metabolic pathway.

As the demand for enzymes with improved activity and novel catalytic function grows, new methods have been developed for isolation of a desired catalyst from a pool of protein variants. Directed evolution has proven particularly successful in cases where enzyme function is directly linked to cell survival, *i.e.,* restoration of an essential activity that has been deleted from an otherwise wild-type cell. Evolution of enzymes that do not themselves provide a selectable phenotype, as in the case of glycosyltransferases (GTases) and other transferases, is much more difficult. Prior to the invention described herein, no method provided a facile or generalized strategy for engineering diverse enzymes.

The isolated biomolecules are purified naturally-occurring, synthetically produced, or recombinant compounds, *e.g.,* polypeptides, nucleic acids, small molecules, or other agents. Purified compounds are at least 60% by weight (dry weight) the compound of interest. Preferably, the preparation is at least 75%, more preferably at least 90%, and most preferably at least 99%, by weight the compound of interest. Purity is measured by any appropriate standard method, for example, by column chromatography, polyacrylamide gel electrophoresis, or HPLC analysis. By "purified" or "substantially purified" is meant a biomolecule or biologically active portion thereof that is substantially free of cellular material or other contaminating macromolecules, *e.g.,* polysaccharides, nucleic acids, or proteins, from the cell or tissue source from which the biomolecule is derived. The phrase "substantially purified" also includes a biomolecule that is substantially free from chemical precursors or other chemicals when chemically synthesized. The language "substantially free of cellular material" includes preparations of biomolecules that are separated from cellular components of the cells from which it is isolated.

### Directed Evolution for Enzyme Engineering and Catalyst Development

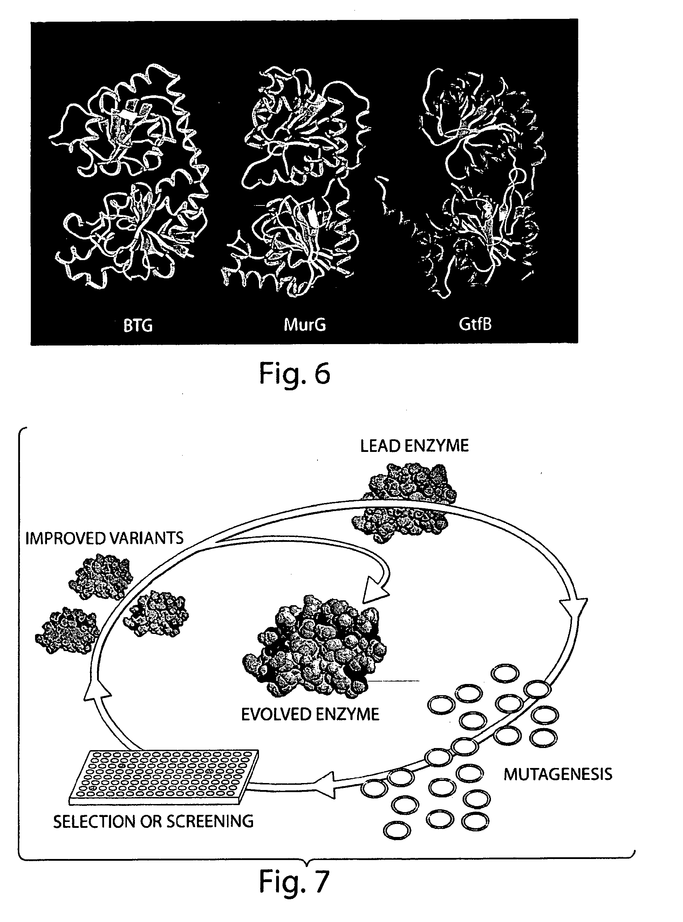

A schematic illustration of the directed evolution for enzyme engineering and catalyst development invention is shown in Figure 7. The invention provides for the ability to coax/generate novel activity from an existing enzyme scaffold by iterative rounds of mutagenesis and selection. As described in detail below, there are many techniques for randomly mutagenizing the desired biomolecule for further selection or screening. There are also many suitable methods for selection and screening. Those skilled in the art will understand that a specific technique can be chosen based on the amount of structural information available for the biomolecule, *e.g.,* protein, of interest. When selecting an individual technique, it is crucial to maintain a link between genotype and phenotype, while maintaining high-throughput.

### Screening Strategy

The invention described here provides an automatable, high-throughput method of evaluating the phenotype of an enzyme encoded by a cell on the basis of multiple parameters, including substrate specificity and kinetic efficiency. This general strategy allows for the *ex vivo* screening of diverse enzymes using native or minimally perturbed substrates. The enzyme of interest is manufactured by the cellular machinery. Alternatively, the disclosure also allows for the screening of other secreted biomolecules, including cytokines, chemokines, antibodies, and metabolites, in solution for a desirable phenotype.

Evaluation of libraries of genetic variants of biomolecules, in particular enzymes, to identify specific members in the library with desired properties (catalytic rate, specificity of reaction, substrate binding affinities) requires both characterizing the phenotype of the biomolecule produced and correlating the biomolecule to the genotype of the member of the library encoding it. In this way, desired variants can be selected and further evaluated. Correlating the phenotype of the biomolecule and the genotype of the producing cell is challenging. The disclosure provides a method for isolating particular members from a library of variant cells in individual microreactors, wherein the phenotype of the biomolecule encoded by the cell is evaluated on the basis of multiple parameters, including substrate specificity and kinetic efficiency. The spatial segregation of the library members allows each to be evaluated in parallel, and members exhibiting desired characteristics are subsequently retrieved for further analysis from the microreactor. A significant application of the technology is the directed evolution of diverse enzymes for use in the *in vitro* construction of biomolecules. One example is a method for the identification of mutant GTases to transfer sugars from activated donor molecules to the appropriate acceptor with absolute chemical control. Such enzymes are capable of competing with chemical synthesis for the rapid and large scale production of complex carbohydrates.

When evolving enzymes from a library of enzyme variants, a simple strategy to link a desirable phenotype to genotype is necessary. The spatial separation of library members in individual compartments allows the identification of variants with unique properties without the requirement of substrate uptake or surface attachment.

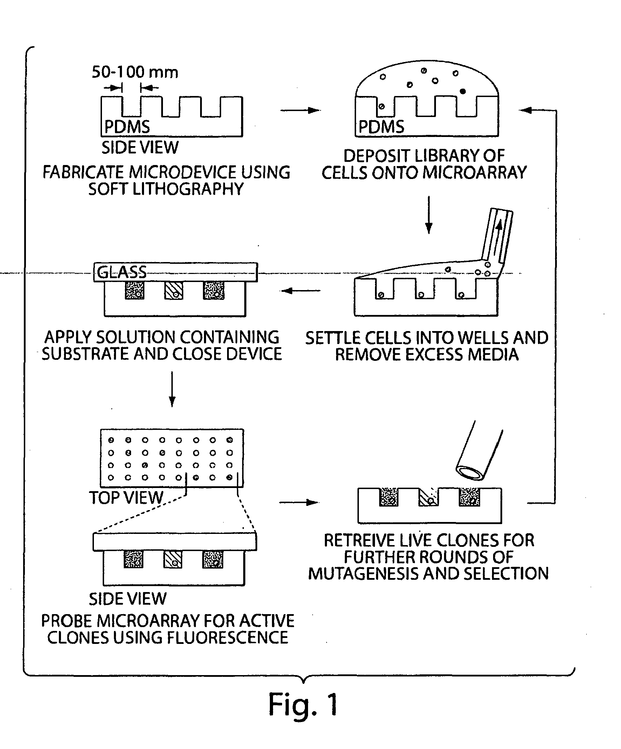

To that end, described herein is the use of microtabricated chambers to separate a library of cells, *e.g.,* yeast cells, which each secrete a mutant version of a protein of interest (Figure 1). The moldable slab, made of poly(dimethylsiloxane), is fabricated by soft lithography and replica molding and is of a biocompatible material, which is not toxic and gas permeable. The rigidity of some materials, such as polystyrene, would not allow for conformal contact, and thus sealing, of the microwells against a substrate for testing the specificity of the antibodies produced in a parallel. PDMS, however, is a suitable material for this technique because it is not toxic, it is gas permeable, and it is easily compressed to form a tight, but reversible, seal with a rigid substrate. Such a seal retards or to prevents any fluid and/or cells in the moldable slab from leaking or escaping.

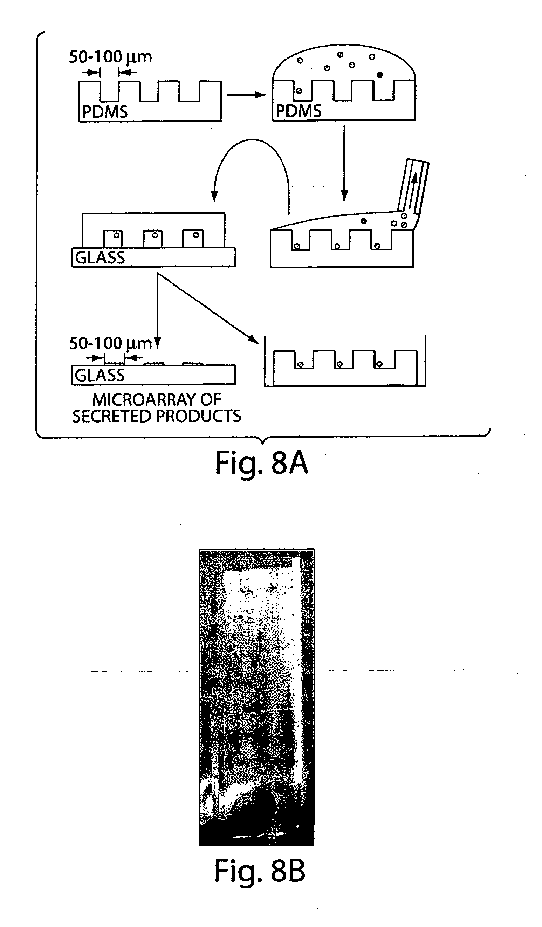

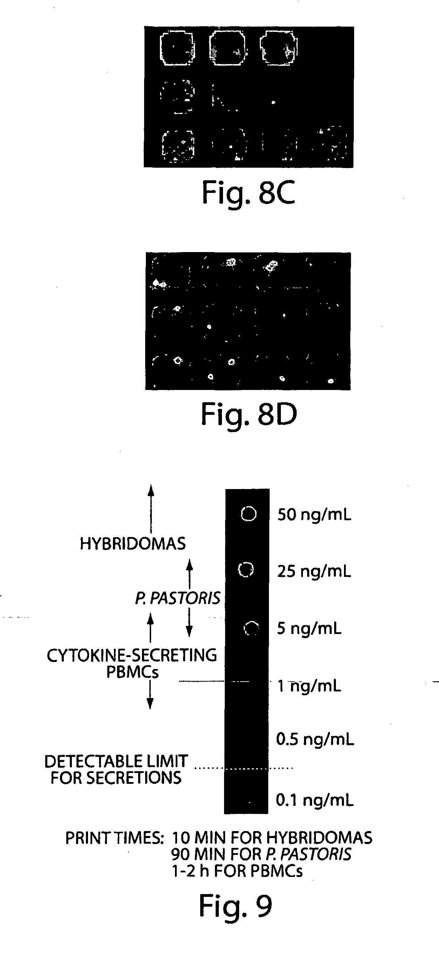

Cells confined in microwells and sealed against a glass slide (such that the total media available was limited to the volume of the microwell) are distributed at roughly one cell per well in a device containing wells 50 µm in diameter. Figure 8C depicts a protein microarray from single *Pichia pastoris* cells and figure 8D shows the cells that secreted the protein microarray in 8C. *Pichia pastoris* cells expressing a human Fc were grown in YPD media overnight. Cells were then loaded into a Poly Dimethyl Siloxane (PDMS) microdevice containing 50 µm wells at roughly one cell per well. The microdevice was contacted with a glass slide pretreated with a goat anti-human Fc antibody to capture the secreted Fc. The secreted proteins were captured over 90 minutes and the resulting array was read using a Cy5-conjugated goat anti-human Ig(H+L) antibody. The *Pichia pastoris* cells were imaged in the microwells using a fluorescent dye for the yeast cell surface. Figure 9 shows how the secereted protein levels for *Pichia pastoris* compare to other cell types, such as hybridomas, and cytokine-secreting peripheral blood mononuclear cells (PBMC). This standard curve was created using purified human Fc, and the intensity values observed were used to assign defined concentrations to the secretions captured from individual cells. The amount of secreted proteins observed for *Pichia pastoris* cells is well above the limit of detection for the assay and should provide adequate concentration levels in microwells for the turnover of supplied enzyme substrates. These experiments demonstrate the ability to detect secreted products from individual yeast cells. *See also*, Love et al., 2006 Nat. Biotechnol, 24(6):703-707; WO 2007/035633.

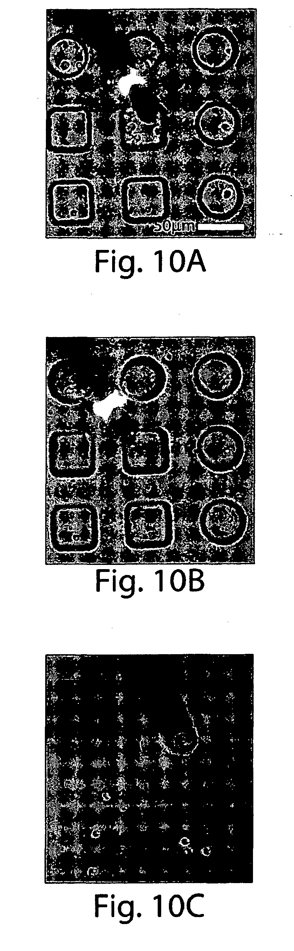

In a particular example, a library of segregated yeast cells is interrogated with enzyme substrates yielding a fluorescence signal upon successful enzyme turnover. Since the intensity of signal correlates directly with product formation, library members are directly compared for enzyme kinetics in addition to substrate specificity via real-time fluorescence monitoring. Clones from fluorescent wells are retrieved using micromanipulation and used in further rounds of evolution and selection. Cell retrieval using a micromanipulator is shown in Figure 10. Yeast survivability following retrieval with a micromanipulator was 40-60%.

### Mutagenesis Techniques for Improving Enzymes

Mutations that encode amino acid changes are useful for generating novel enzyme activities. The genes are obtained using any method known to one of skill in the art, *e.g.,* by isolating clones from a genomic library of a given organism, by polymerase chain reaction (PCR) amplification from a source of genomic deoxyribonucleic acid (DNA) or messenger ribonucleic acid (mRNA), or from a library of expression clones from a heterogeneous mixture of DNA from uncultivated environmental microbes (U.S. Pat. No. 5,958,672). There are numerous methods that are well known to those skilled in the art for mutating the genes encoding enzymes and other non-catalytic proteins and peptides. These methods include both rational (*e.g.,* creating point mutants or groups of point mutants by site-directed mutagenesis) and stochastic (*e.g.,* random mutagenesis, combinatorial mutagenesis and recombination) techniques. A rational design, termed protein design automation, uses an algorithm to objectively predict protein sequences likely to achieve a desired fold.

One class of techniques is those relying on point mutations, *e.g.,* error-prone polymerase chain reaction and oligonucleotide-directed mutagenesis (Cadwell and Joyce, 1992 PCR Methods Applic., 2:28-33; Kegler-Ebo DM, et al., 1994 Nuc Acids Res, 22(9):1593-1599). These methods lead to the production of an enzyme library that contains members having any of the 20 different amino acids at one specific position within a given protein.

Stochastic methods include, for example, chemical mutagenesis (Singer and Kusmierek, 1982 Annu Rev Biochem, 51:655-93), recursive ensemble mutagenesis (Arkin and Youvan, 1992 Proc Natl Acad Sci USA, 89(16):7811-5; Delagrave et al., 1993 Protein Eng, 6(3):327-31), exponential ensemble mutagenesis (Delagrave and Youvan, 1993 Biotechnology, 11(13):1548-52), sequential random mutagenesis (Chen and Arnold, 1991 Biotechnology, 9(11):1073-7; Chen and Arnold, 1993 Proc Natl Acad Sci USA, 90(12):5618-22), DNA shuffling (Stemmer, 1994 Proc Natl Acad Sci USA, 91(22):10747-51; Stemmer, 1994 Nature, 370(6488):389-91) and the like. Recombination is a useful stochastic mutagenesis technique wherein DNA is broken down and rejoined in new combinations. DNA shuffling, the best known method of recombination, allows useful mutations from multiple genes to be combined (Stemmer WPC, et al., 1994 Nature, 370:389-391.) Staggered extension process (StEP) is a simple and efficient method for *in vitro* mutagenesis and recombination of polynucleotide sequences (Zhao H, et al., 1998 Nature Biotechnol, 16:258-261.) Other mutagenesis techniques include chemical mutagenesis and the use of mutator strains (Lai Y, et al., 2004 Biotech Bioeng, 86:622-627; Coia G, et al., 1997 Gene, 201:203-209). These techniques are used individually or in combination to produce mutations.

DNA encoding the desired enzyme or protein is isolated from the expression library and sequenced. By repeating the steps of mutagenesis and screening, novel enzymes and other proteins are artificially created. This iterative process is known as directed evolution. The genes of interest do not necessarily have to be expressed on plasmids. In one aspect, they are expressed following integration into the host chromosome or as a result of mutating the chromosomal copy of a gene. In another aspect, high complexity expression libraries are created without mutagenesis. This can be done by cloning and expressing DNA from a source that already contains a large number of different sequences, such as highly heterogeneous genomic DNA from a mixture of environmental microbes.

### Activity Screening of Expression Libraries

The methods described herein allow for the biomolecule to be assayed for function. In one aspect, screening for the desired biological activity is performed using aptamers, *i.e.,* oligonucleic acid or peptide molecules that bind a specific target molecule. In another aspect, screening for the desired biological activity is performed using a solution-phase FRET-based assay in the microwells of the microdevice with fluorogenic substrates. In another aspect, biological activity is assayed via solid-support fluorescence (or FRET), wherein substrates/products are captured on the contacted glass surface using antibodies. Preferably, one or more of the substrates are fluorescent. In yet another aspect, screening for the desired biological activity is performed via solid-support affinity capture, wherein one or more substrates are further derivitized with a fluorophore using a chemical or enzymatic reaction (*i.e.,* "click chemistry", sortase tagging, BirA biotinylation, etc.). Alternatively, the function of the biomolecule is assayed using a solid-support antibody-based fluorescence readout, wherein both substrates have affinity tags and the product is detected in a sandwich ELISA format. Preferably, the secondary antibody is conjugated to a fluorophore.

In one aspect, screening for the desired biological activity is done by contacting the host cells expressing the enzyme with a chromogenic or fluorogenic compound that is appropriate for the enzyme reaction and monitoring the formation of color in the cells or their surroundings. In the solid-phase assays described in U.S. Pat. No. 5,914,245, these compounds are referred to as optical signal substrates because they produce a measurable change in absorbance, reflectance, fluorescence or luminescence when they come in contact with active enzyme or with a product of the enzymatic reaction.

The disclosure provides for various chromogenic, fluorogenic, lumigenic and fluorescence resonance energy transfer (FRET) substrates to measure biological activity. Typically, fluorophores absorb electromagnetic energy at one wavelength and emit electromagnetic energy at a second wavelength. Representative fluorophores include, but are not limited to, 1,5 IAEDANS; 1,8-ANS; 4-Methylumbelliferone; 5-carboxy-2,7-dichlorofluorescein; 5-Carboxyfluorescein (5-FAM); 5-Carboxynapthofluorescein; 5-Carboxytetramethylrhodamine (5-TAMRA); 5-FAM (5-Carboxyfluorescein); 5-HAT (Hydroxy Tryptamine); 5-Hydroxy Tryptamine (HAT); 5-ROX (carboxy-X-rhodamine); 5-TAMRA (5-Carboxytetramethylrhodamine); 6-Carboxyrhodamine 6G; 6-CR 6G; 6-JOE; 7-Amino-4-methylcoumarin; 7-Aminoactinomycin D (7-AAD); 7-Hydroxy-4-methylcoumarin; 9-Amino-6-chloro-2-methoxyacridine; ABQ; Acid Fuchsin; ACMA (9-Amino-6-chloro-2-methoxyacridine); Acridine Orange; Acridine Red; Acridine Yellow; Acriflavin; Acriflavin Feulgen SITSA; Aequorin (Photoprotein); AFPs--AutoFluorescent Protein--(Quantum Biotechnologies) see sgGFP, sgBFP; Alexa Fluor 350.TM.; Alexa Fluor 430.TM.; Alexa Fluor 488.TM.; Alexa Fluor 532.TM.; Alexa Fluor 546.TM.; Alexa Fluor 568.TM.; Alexa Fluor 594.TM.; Alexa Fluor 633.TM.; Alexa Fluor 647.TM.; Alexa Fluor 660.TM.; Alexa Fluor 680.TM.; Alizarin Complexon; Alizarin Red; Allophycocyanin (APC); AMC, AMCA-S; AMCA (Aminomethylcoumarin); AMCA-X; Aminoactinomycin D; Aminocoumarin; Aminomethylcoumarin (AMCA); Anilin Blue; Anthrocyl stearate; APC (Allophycocyanin); APC-Cy7; APTRA-BTC; APTS; Astrazon Brilliant Red 4G; Astrazon Orange R; Astrazon Red 6B; Astrazon Yellow 7 GLL; Atabrine; ATTO-TAG.TM. CBQCA; ATTO-TAG.TM. FQ; Auramine; Aurophosphine G; Aurophosphine; BAO 9 (Bisaminophenyloxadiazole); BCECF (high pH); BCECF (low pH); Berberine Sulphate; Beta Lactamase; BFP blue shifted green fluorescent protein (GFP) (Y66H); Blue Fluorescent Protein; BFP/GFP FRET; Bimane; Bisbenzamide; Bisbenzimide (Hoechst); bis-BTC; Blancophor FFG; Blancophor SV; BOBO.TM.-1; BOBO.TM.-3; Bodipy 492/515; Bodipy 493/503; Bodipy 500/510; Bodipy 505/515; Bodipy 530/10; Bodipy 542/563; Bodipy 18/568; Bodipy 564/517; Bodipy 576/589; Bodipy 581/591; Bodipy 630/650-X; Bodipy 650/665-X; Bodipy 665/676; Bodipy FI; Bodipy FL ATP; Bodipy FI-Ceramide; Bodipy R6G SE; Bodipy TMR; Bodipy TMR-X conjugate; Bodipy TMR-X, SE; Bodipy TR; Bodipy TR ATP; Bodipy TR-X SE; BO-PRO.TM.-1; BO-PRO.TM.-3; Brilliant Sulphoflavin FF; BTC; BTC-5N; Calcein; Calcein Blue; Calcium Crimson.TM.; Calcium Green; Calcium Green-1 Ca.sup.2+ Dye; Calcium Green-2 Ca.sup.2+; Calcium Green-5N Ca.sup.2+; Calcium Green-C18 Ca.sup.2+; Calcium Orange; Calcofluor White; Carboxy-X-rhodamine (5-ROX); Cascade Blue.TM.; Cascade Yellow; Catecholamine; CCF2 (GeneBlazer); CFDA; CFP--Cyan Fluorescent Protein; CFP/YFP FRET; Chlorophyll; Chromomycin A; Chromomycin A; CL-NERF; CMFDA; Coelenterazine; Coelenterazine cp; Coelenterazine f; Coelenterazine fcp; Coelenterazine h; Coelenterazine hep; Coelenterazine ip; Coelenterazine n; Coelenterazine O; Coumarin Phalloidin; C-phycocyanine; CPM Methylcoumarin; CTC; CTC Formazan; Cy2.TM.; Cy3.1 8; Cy3.5.TM.; Cy3.TM.; Cy5.1 8; Cy5.5.TM.; Cy5.TM.; Cy7.TM.; Cyan GFP; cyclic AMP Fluorosensor (FiCRhR); Dabcyl; Dansyl; Dansyl Amine; Dansyl Cadaverine; Dansyl Chloride; Dansyl DHPE; Dansyl fluoride; 4',6-diamidino-2-phenylindole (DAPI); Dapoxyl; Dapoxyl 2; Dapoxyl 3' DCFDA; DCFH (Dichlorodihydrofluorescein Diacetate); DDAO; DHR (Dihydorhodamine 123); Di-4-ANEPPS; Di-8-ANEPPS (non-ratio); DiA (4-Di-16-ASP); Dichlorodihydrofluorescein Diacetate (DCFH); DiD-Lipophilic Tracer; DiD (DiIC18(5)); DIDS; Dihydorhodamine 123 (DHR); Dil (DilC18(3)); Dinitrophenol; DiO (DiOC18(3)); DiR; DiR (DiIC18(7)); DM-NERF (high pH); 2,4-Dinitrophenol (DNP); Dopamine; DsRed; DTAF; DY-630-NHS; DY-635-NHS; EBFP; ECFP; EGFP; ELF 97; Eosin; Erythrosin; Erythrosin ITC; Ethidium Bromide; Ethidium homodimer-1 (EthD-1); Euchrysin; EukoLight; Europium (III) chloride; EYFP; Fast Blue; FDA; Feulgen (Pararosaniline); FIF (Formaldehyd Induced Fluoreseence); FITC; Flazo Orange; Fluo-3; Fluo-4; Fluorescein (FITC); Fluorescein Diacetate; Fluoro-Emerald; Fluoro-Gold (Hydroxystilbamidine); Fluor-Ruby; FluorX; FM 1-43.TM.; FM 4-46; Fura Red.TM. (high pH); Fura Red.TM./Fluo-3; Fura-2; Fura-2/BCECF; Genacryl Brilliant Red B; Genacryl Brilliant Yellow 10GF; Genacryl Pink 3G; Genacryl Yellow 5GF; GeneBlazer (CCF2); GFP (S65T); GFP red shifted (rsGFP); GFP wild type, non-UV excitation (wtGFP); GFP wild type, UV excitation (wtGFP); GFPuv; Gloxalic Acid; Granular blue; Haematoporphyrin; Hoechst 33258; Hoechst 33342; Hoechst 34580; HPTS; Hydroxycoumarin; Hydroxystilbamidine (FluoroGold); Hydroxytryptamine; Indo-1, high calcium; Indo-1, low calcium; Indodicarbocyanine (DiD); Indotricarbocyanine (DiR); Intrawhite Cf; JC-1; JO-JO-I; JO-PRO-1; LaserPro; Laurodan; LDS 751 (DNA); LDS 751 (RNA); Leucophor PAF; Leucophor SF; Leucophor WS; Lissamine Rhodamine; Lissamine Rhodamine B; Calcein/Ethidium homodimer; LOLO-1; LO-PRO-1; Lucifer Yellow; Lyso Tracker Blue; Lyso Tracker Blue-White; Lyso Tracker Green; Lyso Tracker Red; Lyso Tracker Yellow; LysoSensor Blue; LysoSensor Green; LysoSensor Yellow/Blue; Mag Green; Magdala Red (Phloxin B); Mag-Fura Red; Mag-Fura-2; Mag-Fura-5; Mag-Indo-1; Magnesium Green; Magnesium Orange; Malachite Green; Marina Blue; Maxilon Brilliant Flavin 10 GFF; Maxilon Brilliant Flavin 8 GFF; Merocyanin; Methoxycoumarin; Mitotracker Green FM; Mitotracker Orange; Mitotracker Red; Mitramycin; Monobromobimane; Monobromobimane (mBBr-GSH); Monochlorobimane; MPS (Methyl Green Pyronine Stilbene); NBD; NBD Amine; Nile Red; Nitrobenzoxadidole; Noradrenaline; Nuclear Fast Red; Nuclear Yellow; Nylosan Brilliant lavin E8G; Oregon Green; Oregon Green 488-X; Oregon Green.TM.; Oregon Green.TM. 488; Oregon Green.TM. 500; Oregon Green.TM. 514; Pacific Blue; Pararosaniline (Feulgen); PBFI; PE-Cy5; PE-Cy7; PerCP; PerCP-Cy5.5; PE-TexasRed [Red 613]; Phloxin B (Magdala Red); Phorwite AR; Phorwite BKL; Phorwite Rev; Phorwite RPA; Phosphine 3R; PhotoResist; Phycoerythrin B[PE]; Phyeoerythrin R [PE]; PKH26 (Sigma); PKH67; PMIA; Pontochrome Blue Black; POPO-1; POPO-3; PO-PRO-1; PO-PRO-3; Primuline; Procion Yellow; Propidium Iodid (PI); PyMPO; Pyrene; Pyronine; Pyronine B; Pyrozal Brilliant Flavin 7GF; QSY 7; Quinacrine Mustard; Red 613 [PE-TexasRed]; Resorufin; RH 414; Rhod-2; Rhodamine; Rhodamine 110; Rhodamine 123; Rhodamine 5 GLD; Rhodamine 6G; Rhodamine B; Rhodamine B 200; Rhodamine B extra; Rhodamine BB; Rhodamine BG; Rhodamine Green; Rhodamine Phallicidine; Rhodamine Phalloidine; Rhodamine Red; Rhodamine WT; Rose Bengal; R-phycocyanine; R-phycoerythrin (PE); rsGFP; S65A; S65C; S65L; S65T; Sapphire GFP; SBFI; Serotonin; Sevron Brilliant Red 2B; Sevron Brilliant Red 4G; Sevron Brilliant Red B; Sevron Orange; Sevron Yellow L; sgBFP.TM.; sgBFP.TM. (super glow BFP); sgGFP.TM.; sgGFP.TM. (super glow GFP); SITS; SITS (Primuline); SITS (Stilbene Isothiosulphonic Acid); SNAFL calcein; SNAFL-1; SNAFL-2; SNARF calcein; SNARF1; Sodium Green; SpectrumAqua; SpectrumGreen; SpectrumOrange; Spectrum Red; SPQ (6-methoxy-N-(3-sulfopropyl)quinolinium); Stilbene; Sulphorhodamine B can C; Sulphorhodamine Extra; SYTO 11; SYTO 12; SYTO 13; SYTO 14; SYTO 15; SYTO 16; SYTO 17; SYTO 18; SYTO 20; SYTO 21; SYTO 22; SYTO 23; SYTO 24; SYTO 25; SYTO 40; SYTO 41; SYTO 42; SYTO 43; SYTO 44; SYTO 45; SYTO 59; SYTO 60; SYTO 61; SYTO 62; SYTO 63; SYTO 64; SYTO 80; SYTO 81; SYTO 82; SYTO 83; SYTO 84; SYTO 85; SYTOX Blue; SYTOX Green; SYTOX Orange; Tetracycline; Tetramethylrhodamine (TRITC); Texas Red.TM.; Texas Red-X.TM. conjugate; Thiadicarbocyanine (DiSC3); Thiazine Red R; Thiazole Orange; Thioflavin 5; Thioflavin S; Thioflavin TCN; Thiolyte; Thiozole Orange; Tinopol CBS (Calcofluor White); TMR; TO-PRO-1; TO-PRO-3; TO-PRO-5; TOTO-1; TOTO-3; TriColor (PE-Cy5); TRITC TetramethylRodaminelso ThioCyanate; True Blue; TruRed; Ultralite; Uranine B; Uvitex SFC; wt GFP; WW 781; X-Rhodamine; XRITC; Xylene Orange; Y66F; Y66H; Y66W; Yellow GFP; YFP; YO-PRO-1; YO-PRO-3-; YOYO-1-; YOYO-3, Sybr Green, Thiazole orange (interchelating dyes), semiconductor nanoparticles such as quantum dots, or caged fluorophore (which can be activated with light or other electromagnetic energy source) or a combination thereof.

A wide variety of suitable donor (D) and acceptor (A) fluorophores suitable for use in FRET are commercially available. The choice of probe pair is influenced by system constraints as well as by the length and sequence of the peptide used in the desired application. The length and sequence of the peptide will influence the labeling sites for attachment of the probes. The distance between the attachment sites influences the choice of the donor/acceptor pair due to the distance-dependence of FRET. Many donor/acceptor pairs are commercially available. These include, but are not limited to: 5-TAMRA/QSY-7; Dansyl / Eosin; Tryptophan / Dansyl; Fluorescein / Texas Red (rhodamine); Naphthalene/Dansyl; Dansyl / ODR; BODIPY / BODIPY; Terbium / Thodamine; Dansyl / FITC; Pyrere / Coumarin; IAEDANS / IAFBPE / Cy5; and Europium / Cy5. A biotin or other small affinity tag is used in detection of the protein via anti-biotin antibodies or avidin/streptavidin tagged detectors like horseradish peroxidase or a fluorescent dye.

In one aspect, indicator compounds are used to detect one or more products of an enzymatic reaction by interacting either directly or indirectly with the products. Optionally, these indicator compounds are included as part of the optical signal substrate solution. For example, U.S. Pat. No. 5,914,245 describes a lipase assay that detects fatty acid interactions with the fluorescent dye Rhodamine B. Other assays that can utilize indicator compounds include those wherein protons are generated or wherein transmembrane proton, electron or ion transfer occurs during an enzymatic reaction. These activities can be detected by including various dyes in the substrate solution. Fluorescein isothiocyanate (FITC) is a derivative of fluorescein used in wide ranging applications including flow cytometry. Exemplary fluorescent indicator dyes used to monitor pH changes include fluorescein and seminaphthorhodafluors and their derivatives for the pH range 6-9 and LysoSensor, Oregon Green and Rhodol and their derivatives for the pH range 3-7. These fluorescent pH indicators are available from Molecular Probes (Eugene, Oreg.). Chromophore dyes whose wavelength of maximum absorption changes as a function of pH include Thymol Blue (approximate useful pH range 1.2-2.8 and 8.0-9.6), Methyl Orange (pH 3.2-4.4), Bromocresol Green (pH 3.8-5.4), Methyl Red (pH 4.2-6.2), Bromothymol Blue (pH 6.0-7.6) and Phenol Red (pH 6.8-8.2). Phenolphthalein (pH 8.2-10.0) turns from colorless to pink as the pH becomes more alkaline. These colorimetric pH indicators are available from Sigma-Aldrich (St. Louis, Mo.). There are numerous examples in enzymology of using pH indicators for detecting enzymatic activity (Lowry et al., 1951 J Biol Chem, 193:265-275; Khalifah, 1971 J Biol Chem, 246(8):2561-73). Indicators such as Bromothymol Blue and Phenol Red have been used to assay the activity of various hydrolases in solution (Moris-Varas et al., 1999 Bioorg Med Chem, (10):2183-8).

### Mucin-Type O-Linked Glycosylation

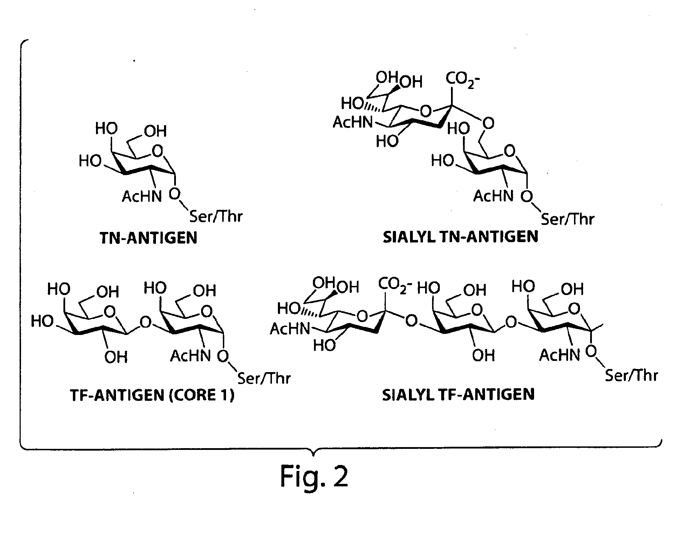

The most abundant form of O-linked glycosylation in higher eukaryotes is known as "mucin-type" (Hang H and Bertozzi C, 2005 Bioorg Med Chem, 13(17):5021-5034). The first step in mucin biosynthesis is α-*N*-acetylgalactosamine (GalNAc) addition to hydroxyl groups of serine or threonine side chains to form the Tn-antigen; this transfer is accomplished by the polypeptide *N*-acetyl-α-galactosaminyltransferases (ppGalNAcTases) (Ten Hagen et al., 2003 Glycobiol, 13(1):1R-16R). The Tn-antigen is elaborated further by downstream GTases to produce a variety of mucin-type structures (Figure 2). To date, over 150 glycoproteins containing mucin-type glycosylation have been identified, many of which are involved in disease progression (Hang 2005). One such example is MUC1, a glycoprotein that has been identified as a tumor antigen due to its increased expression in cancer epithelial cells, which contributes to both cancer cell adhesion and tumor invasiveness (Yu et al., 2007 J Biol Chem, 282(1):773-781; Kohlgraf et al., 2003 Cancer Res, 63(16):5011-5020). Cancer-associated mucins are highly immunogenic and may be used as targets for immunotherapy (Hanisch and Ninkovic, 2006 Curr Prot Pep Sci, 7:307; Tarp and Clausen, In Press Biochem Biophys Acta).

### Synthesis of Homogeneous Mucin-Type O-Linked Glycopeptides and Glycoproteins

The development of carbohydrate vaccines requires access to large quantities of homogeneous glycopeptides and glycoproteins (Grogan et al., 2002 Annu Rev Biochem, 71:593-634). The isolation of native or recombinant glycoproteins, however, only yields limited amounts of heterogeneous glycoforms, each of which can display different biological properties (Freire et al., 2006 Glycobiol, 16(11):1150). Chemical synthesis of glycoconjugates provides homogeneous substrates via solid-phase peptide synthesis (SPPS) using an appropriately protected glycosyl amino acid building block (Marcaurelle and Bertozzi, 2002 Glycobiol, 12(6):69R-77R). Native chemical ligation and expressed protein ligation have also been used to install sugars site-specifically in larger peptides and even proteins (Muir TW, 2003 Annu Rev Biochem, 72:249-289). Prior to the invention described herein, accomplishing these synthetic methods still required a specially trained chemist. The invention provides for the generation of enzymes capable of efficient synthesis of glycoconjugates on a preparative scale, which greatly aids in their study for therapeutic purposes.

### GTase Evolution for the Synthesis of Carbohydrate-Containing Natural Products.

The identification of glycoproteins and glycolipids that are overexpressed on the surfaces of cancer cells has led to their investigation as targets for immunotherapy (Slovin et al., 2005 Immunol Cell Biol, 83(4):418). As tumor associated carbohydrate antigens are typically expressed in low levels and in various glycoforms, the isolation of sufficient amounts of discrete glycoconjugates for developing carbohydrate-based anticancer vaccines is difficult. Prior to the invention described herein, general methods for the chemical synthesis of carbohydrates have improved with the advent of automated assembly (Plante et al., 2003 In: Advances in Carbohydrate Chemistry and Biochemistry, Vol. 58, pp 35-54), but still require a specialist to accomplish the extensive protecting group manipulations requisite for stereochemical control and donor/acceptor compatibility. Additional shortcomings of the chemical synthesis of glycoconjugates include the difficulty in generating large scale amounts to meet clinical requirements and the difficulty in purifying the synthesized materials.

Nature efficiently makes carbohydrate-containing compounds using glycosyltransferases (GTases) to transfer sugars from activated donor molecules (*e.g.,* UDP sugars) to the appropriate acceptor (*e.g.,* proteins/peptides, lipids, other sugars and natural product aglycones - polyketides and macrolides) with absolute chemical control/stereochemistry. The following GTases glycosylate diverse acceptors using three different donors, yet have a very similar fold: GtfB - glucose transfer to vancomycin aglycone, BTG β-glucosyl transferase, and MurG - GlcNAc transfer in cell wall biosynthesis.

While most GTases are highly substrate selective, relatively few structural motifs are used to glycosylate a wide range of glycosyl acceptors (Hu Y and Walker S, 2002 Chem Biol, 9:1287-1296). The invention provides for directed evolution of existing GTases to identify more potent catalysts with altered substrate selectivity. More specifically, the invention provides for the identification of mutant GTases capable of competing with chemical synthesis for the rapid and large scale production of glycoconjugates for therapeutic purposes, including carbohydrate based cancer vaccines.

Engineered GTases have enormous potential for the synthesis of biologically relevant glycoconjugates, either by improving the catalytic efficiency of native glycosylation or by incorporating non-natural sugar residues (Hancock et al., 2006 Curr Opin Chem Biol, 10(5):509-519). However, prior to the invention described here, few attempts had been made to engineer GTases by directed evolution, largely due to the lack of methods for screening and selecting mutants on the basis of GTase activity. Recent examples include the engineering of a sialylotransferase (Lairson et al., 2006 Nat. Chem. Biol., 2(12):724-728) and a glucotransferase (Williams et al., 2007 Nat. Chem. Biol., 3(10):657-662), but the generality of the screening methods used in these cases is unclear. The first method requires fluorescent substrates to be ingested by competent clones to sort them by flow cytometry, and the second method uses the fluorescent molecules themselves as the aglycone acceptors. The invention described here provides a general strategy that allows for the *ex vivo* screening of diverse enzymes using native or minimally perturbed substrates.

M13 phage display is a convenient strategy to link phenotype and genotype in the engineering and selection of enzymes that do not provide cell-based phenotypes (Hoess R, 2001 Chem Rev, 101:3205-3218). In phage-display enzyme evolution, enzymes and substrates are proximally bound on the surface of phage to enable deconvolution of the library by affinity capture of the products. Recently, a chemically straightforward method was developed for the attachment of substrates to the surface of phage using selenocysteine residues (Love et al., 2006 Chembiochem, 7(5):753-756). In that study, the bacterial GTase MurG was expressed on phage in active form; however, a successful evolution of MurG was unsuccessful due to the inability of phage-bound enzyme to utilize phage-bound substrate. The new technique provided by the invention extends the method to eukaryotic enzymes and provides improved methods of screening a library of mutant GTases.

One advantage of the methods described by the invention is that neither the enzymes to be assayed, nor the substrates for those enzymes need to be attached to any type of solid support, *e.g.,* a solid surface, another cell, etc. Moreover, the methods of the invention are performed in solution with secreted biomolecules. The invention provides for screening biomolecules secreted from individual cells, instead of from microcolonies, which are clumps of cells. Additionally, cells secreting active clones are retrieved from the device by micromanipulation with a glass capillary, and then either mutagenized randomly for further selection or sequenced to identify the encoded enzyme.

Another distinguishing characteristic of the methods described by the invention is that multiple characteristics of each library member are assessed during the screening process. Unlike surface-display methods on phage, bacterial cells or yeast, the rates of enzymatic turnover can be monitored in real-time in the microreactors on the basis of changes in the measured fluorescent intensities. Competitive assays using two substrates modified with different fluorophores allows direct monitoring of substrate specificity or selectivity during the screening. These measurements provide a greater degree of diversity in the clones identified and selected for further rounds of evolution than existing techniques.

### Applications

Biocatalysis is an important tool for the synthesis of bulk chemicals, pharmaceuticals and food ingredients. The number and diversity of such applications are limited, however, likely due to limitations in enzyme stability, catalytic properties, *i.e.,* turnover rate, and substrate scope. Access to a tool kit of biocatalysts will help industry overcome the current limitations and enable the realization of many new applications, from single-step enzymatic conversion to multi-step microbial synthesis via metabolic pathway engineering.

The biosynthesis of carbohydrate-containing natural products is of particular interest in industry, as their synthesis by traditional means requires lengthy protecting group manipulations and studies in glycosyl donor/acceptor compatibility. Therapeutic vaccines derived from glycoprotein or glycolipid constructs that are overexpressed on the surfaces of malignant cells are a promising approach for cancer immunotherapy.

### Synthesis of Novel Macrolide Antibiotics

The increasing incidence of antibiotic resistant bacterial infections indicates the need for improved constructs to treat enterococcal-infected patients. Many macrolide and polyketide antibiotics contain carbohydrates that participate in recognition of a cellular target and are thereby essential for activity (Walsh C, 2003 Antibiotics: Actions, origins, resistance. 1st ed.; American Society for Microbiology Press: Washington, D.C.). Modification of existing glycopeptide antibiotics, such as vancomycin and teicoplanin, on and around the sugar substitutents has led to the clinical trials of new treatments, including oritavancin (Dong et al., 2002 J Am Chem Soc, 124:9064-9065). Adaptation of GTases as catalysts for the attachment of diverse carbohydrates to natural product aglycones, proteins and lipids will provide new materials for investigation as therapeutic agents. The methods provided by the invention identify enzymes capable of efficiently glycosylating a range of substrates and segue into the generation of catalysts able to compete with chemical synthesis for the rapid and large scale production of glycoconjugates.

### Example 1. The Development of a New Technique For Screening a Library of Mutant Enzymes For Improved Catalytic Activity or Altered Substrate Specificity

The following experiment consists of (1) illustration of a technique for the spatial separation of a library of yeast cells secreting an enzyme of interest, and (2) enrichment of cells expressing an active protease from an inactive variant to determine the sensitivity of the technique. Briefly, a library of yeast cells capable of secreting a protein of interest is loaded into microwells 50 microns in diameter so that each well contains, on average, one library member. Each compartment in the device is interrogated in parallel with enzyme substrates; successful enzyme turnover yields a fluorescence signal. Feasibility of the technique is demonstrated with a protease.

Microfabricated arrays of wells have been used for diverse biological applications. Microwells have proven useful to study enzymology at the single molecule level, and wells that are 50-100 µm diameter have been used to separate cells to screen secreted products captured on a surface (Rondelez et al., 2005 Nat Biotechnol, 23(3):361-365; Love et al., 2006 Nat Biotechnol, 24(6):703-707). Microdevices of the latter sort contain ∼100,000 wells on a footprint the size of a typical microscope slide (1" × 3") making screening of a reasonably sized library (10⁶ members) possible using 10 such devices in one day on an optical microscope.

### Selection of Expression Host

The disclosure provides for screening biomolecules secreted by cells. In one aspect, the cells are prokaryotic cells. Alternatively, the cells are eukaryotic cells. Preferably, the eukaryotic cells are yeast cells. An exemplary yeast cell includes *Pichia pastoris.* Yeast cells that secrete plasmid encoded proteins are used for the expression of enzymes for evolution by means of the methods of the invention. Eukaryotic expression hosts, such as yeast, offer an advantage over bacterial expression for the evolution of diverse enzymes, including the ppGalNAcTases, because they contain the machinery necessary for proper protein folding, secretion and post-translational modification. Yeast are also an ideal size (∼5-10 µm in diameter) for spatial separation using microdevices in a ratio of one cell per well, where each well is 50 µm in diameter. Additionally, yeast divide rapidly making the genotyping of a library member derived from a single cell possible within hours.

Yeast cells capability to secrete encoded enzymes vary with respect to cell cycle; yeast are most efficient at protein secretion during the budding process. In one aspect, large variations in secretion, or the inability of the yeast to secret a particular protein of interest is circumvented using yeast surface display. Yeast surface display has been useful in the evolution of diverse antibodies-and several active enzymes have been previously displayed on the surface of yeast (Gai and Wittrup, 2007 Current Opinion in Structural Biology, 17(4):467-473).

### Validation of the Technique With a Model Enzyme

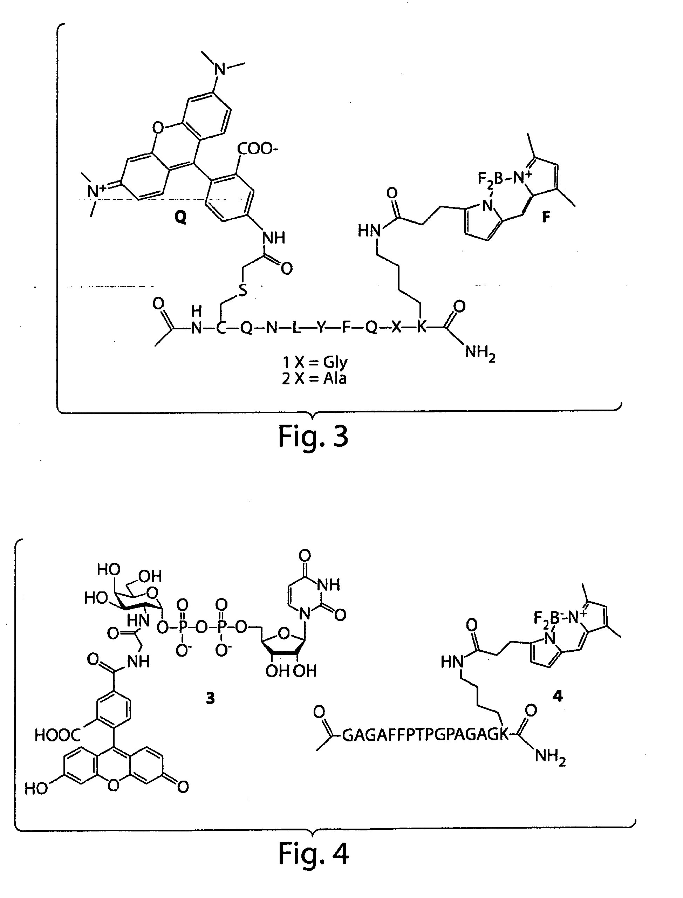

The feasibility of the devised enzyme selection strategy is tested first with the 3C-type cysteine protease from tobacco etch virus (TEV) (Malcolm B, 1995 Protein Sci, 4(8):1439-1445). Mutation of the catalytic cysteine in TEV at residue 151 to alanine results in a catalytically inactive variant (Phan et al., 2002 J Biol Chem, 277(52):50564-50572). Vectors containing the genes for native and mutant species of TEV protease are mixed in various ratios (1:10,000, 1:1000, 1:100, 1:10) and used to create a model library for enrichment of the catalytically active species. Yeast cells transformed with the vector mixture are segregated into wells as outlined above (Figure 1). Sensitivity of the assay is determined using the optimum recognition site (ENLYFQG; SEQ ID NO: 1) for TEV protease as part of a fluorescence resonance energy transfer (FRET) substrate (option 1; Figure 3) (Malcolm 1995; Behlke *et al.,* 2005, Fluorescence and fluorescence applications. Integrated DNA Technologies). Peptide cleavage between the glutamine and glycine residues disrupts the intramolecular FRET quenching and result in a fluorescence signal.

### Evolution of Catalytic Activity and Substrate Specificity

Following the successful enrichment of clones expressing active catalysts, model experiments for the directed evolution of the TEV protease are conducted. To further demonstrate the ability to screen on the basis of catalytic activity, the inactive C151A mutant is randomly mutagenized using error-prone PCR (polymerase chain reaction) to recover catalytically competent variants. While activity will likely be restored as a result of the direct inversion of the mutation at residue 151, it is possible to identify competent variants with alternate mutations. As the ability to screen for enzyme kinetics is anticipated, it is possible to identify clones with increased catalytic activity as compared to wild-type TEV protease. Finally, a library of variants constructed from mutagenesis of the wild-type TEV protease for cleavage of a non-native substrate is examined (option 2 (2, X = Ala) Figure 3). After each round of selection, cells secreting active clones are retrieved from the device by micromanipulation with a glass capillary. Retrieved clones will either be randomly mutagenized for further selection or sequenced to identify the encoded enzyme.

### Example 2. The Evolution of a Mutant GTase With Improved Catalytic Activity

The following experiment consists of evolution of ppGalNAcTase mutants with increased catalytic efficiency and altered substrate specificity. Microdevices are used to screen for mutants of ppGalNAcTase-T1 having improved catalytic efficiency. ppGalNAcTase-T1 is responsible for the transfer of alpha-GalNAc to Ser/Thr residues to form the Tn-antigen - a tumor-associated carbohydrate epitope. Mutants identified in this screen are used for the *in vitro* synthesis of the Tn-antigen.

A recent crystal structure of murine ppGalNAcTase-T1 shows that this protein folds to form distinct catalytic and lectin domains (Fritz et al., 2004 Proc Natl Acad Sci, 101(43):15307-15312). Error-prone PCR is used to create random libraries of ppGalNAcTase-T1 mutagenized within the catalytic domain. A library of transformants is spatially segregated as previously described and screened using fluorescent substrates (Figure 4).

### Design and Synthesis of Fluorescent ppGalNAcTase Substrates

A fluorescein-modified UDP-sugar donor along with a TAMRA-modified peptide acceptor allows for product detection at 580 nm due to FRET between the two fluorophores following glycosylation (Behlke 2005). Based on structural information about the UDP-sugar binding pocket of ppGalNAcTase-T1 and other retaining GTases, a UDP-GalNAc substrate (**3**) bearing fluorescein at C-2 is synthesized as previously reported for UDP-GlcNAc (Fritz 2004; Patenaude 2002; Helm et al., 2003 J Am Chem Soc, 125:11168-11169). Acceptor peptide **4** containing an optimized substrate sequence (GAGAFFPTPGPAGAGK; SEQ ID NO: 2) for glycosylation by ppGalNAcTase-T1 is synthesized with a C-terminal TAMRA using commercially available reagents (Gerken et al., 2006 J Biol Chem, 281(43):32403-32416).

### Confirmation of Activity in Retrieved Clones

Following adequate rounds of library selection and amplification (typically 4-6), cells secreting active clones are retrieved from the device by micromanipulation with a glass capillary, and then either mutagenized randomly for further selection or sequenced to identify the encoded enzyme. Encoded enzymes are tested with the native, unmodified UDP-GalNAc and peptide substrates to identify those best able to synthesize the Tn-antigen *in vitro.* Capable library members are used to synthesize Tn-antigen in large quantities for further study of its immunological properties and potential use in developing anticancer vaccines.

Secreted or surface-displayed enzymes may not be capable of utilizing synthetic substrates containing bulky fluorophores incorporated to assay enzyme function. In one aspect, the position of the fluorophores within each substrate, particularly the modified UDP-GalNAc, are changed until an accepted version is achieved. Alternatively, azido-functionalized UDP sugars are routinely employed to study glycosylation *in vivo;* the azide group is a useful chemical tag for further derivatization and substrate detection (Campbell et al., 2007 Molecular Biosystems, 3(3):187-194). In another aspect, the ppGalNAcTase acceptor peptide is modified with biotin to allow for capture and subsequent detection of coupled products with a lectin or antibody in a sandwich-style assay. In one aspect, the biotin tag is used in affinity chromatography together with a column that has avidin (also streptavidin or Neutravidin) bound to it, which is the natural chelator for biotin. Alternatively, this tag is used in detection of the protein via anti-biotin antibodies or avidin/streptavidin tagged detectors like horseradish peroxidase or a fluorescent dye.

### Evolution of a Mutant ppGalNAcTase With Altered Substrate Preference

Structural studies of a retaining glycosyltransferase closely related to ppGalNAcTase-T1 have shown that specific residues of the enzyme contact moieties in the UDP-sugar donor (C-3 and C-4) to enhance specificity for UDP-GalNAc over UDP-GlcNAc (Patenaude et al., 2002 Nat Struct Biol, 9(9):685-690; Fritz et al., 2006 J Biol Chem, 281(13):8613-8619). Screening the library of mutagenized T1 variants described above with a fluorescein-modified UDP-GlcNAc donor yields clones capable of transferring this non-native substrate and improves the understanding of the active-site specificity of GTases.

### Extension to the Synthesis of Other Mucin-Type Glycoconjugates

The synthesis described above is extended by mutagenizing sialyl transferase ST6GalNAc-1 to make the sialyl Tn-antigen (Figure 2), using the *in vitro* synthesized Tn-antigen as a substrate. Development of enzymes for the *in vitro* synthesis of various mucin-type core structures enables the biological study of this class of glycoconjugates, which have been implicated in a variety of diseases.

### Example 3. Enzyme Turnover in Microwells - Trypsin Cleavage Assay

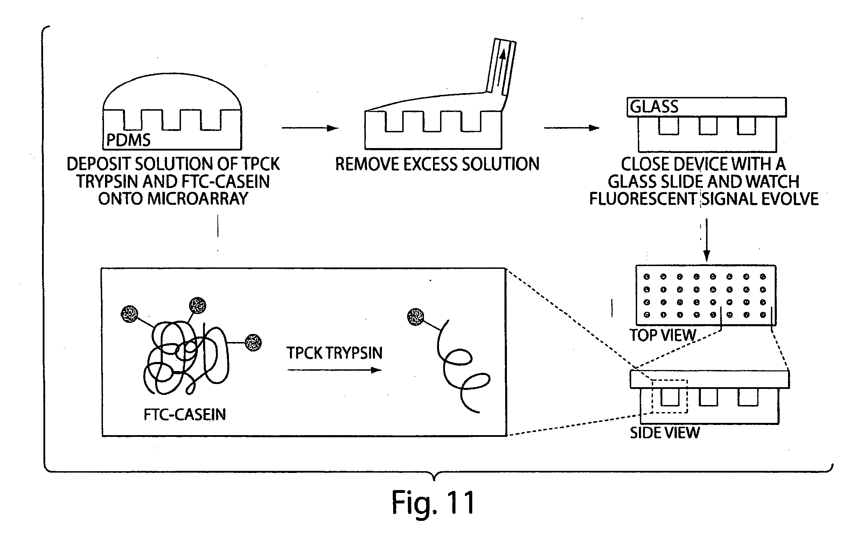

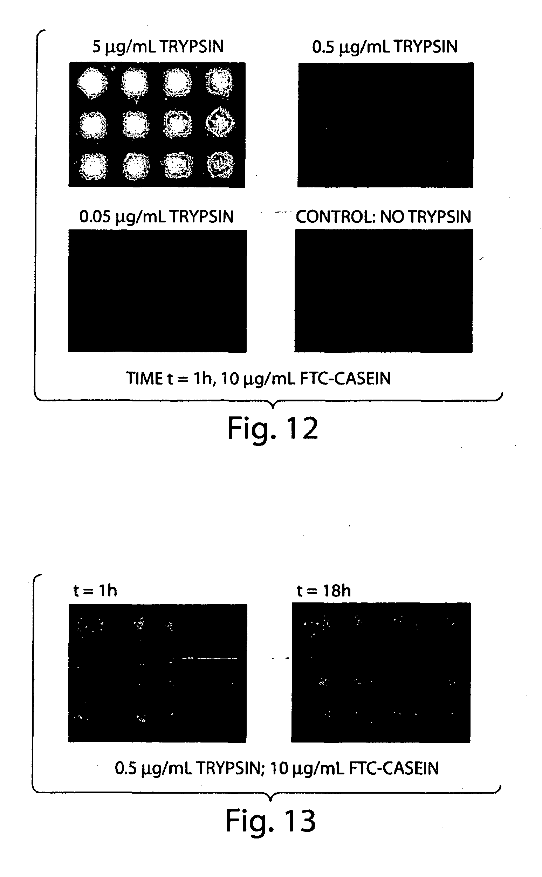

The following experiment demonstrates detection of enzyme activity in a cell-free microwell system. A method for detecting enzyme turnover in microwells via a trypsin cleavage assay is diagramed in Figure 11. Increasing concentrations (0.05 µg/ml, 0.5 µg/ml, and 5 µg/ml) of trypsin were incubated with 10 µg/ml FTC-casein for 1 hour in microwells. As shown in Figure 12, the intensity of the observed fluorescent signal was dependent on the concentration of trypsin in the microwells. In a separate experiment, 0.5 µg/ml of trypsin was incubated with 10 µg/ml FTC-casein in microwells, and photomicrographs were taken at 1 and 18 hours. As shown in Figure 13, the intensity of the observed fluorescent signal was dependent on the time of incubation. Microwells have been used previously to study isolated enzymes in microwells. *See,* JP2004309405A1; and Rondelez et al., 2005 Nat Biotechnol, 23(3):361-365.

### Example 4. Secreted Enzyme Turnover in Microwells - HRV-3C Protease Assay

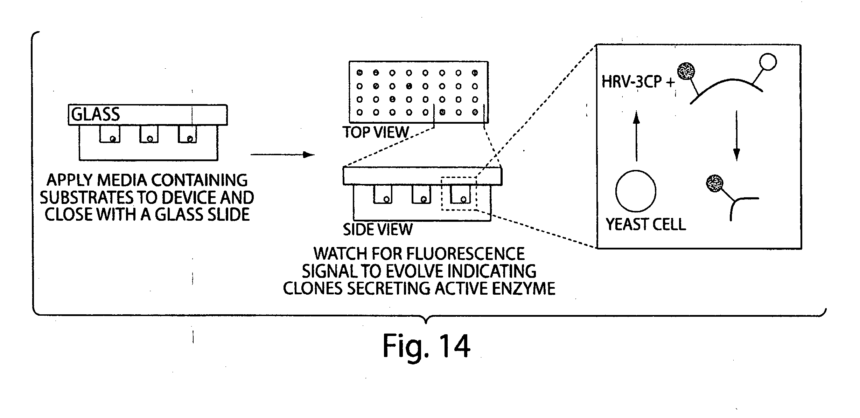

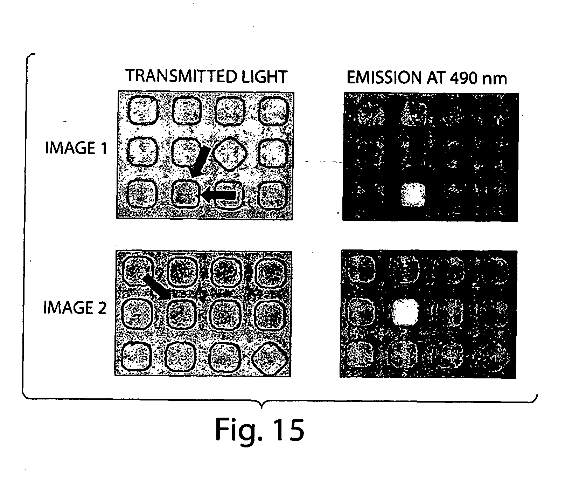

The following experiment demonstrates that an enzyme, *i.e.,* a protease secreted by individual *Pichia pastoris* (yeast) cells inside the micro-device of the invention, cleaved a peptide substrate with a FRET reporter pair, thereby identifying cells containing active enzyme with a bright fluorescent signal. Specifically, *Pichia pastoris* were genetically engineered to secrete human rhinovirus 3C protease (HRV-3CP), which cleaved a peptide substrate sequence (EDANS-A-L-E-V-L-F-Q/G-P-K-DABCYL; SEQ ID NO: 3). A method for detecting enzyme turnover in microwells via an HRV-3CP assay is diagramed in Figure 14. Pichia pastoris capable of secreting the HRV-3CP enzyme were loaded into the microdevice. The cells were incubated in the microdevice for 18 hours in the presence of the FRET peptide substrate (EDANS-A-L-E-V-L-F-Q/G-P-K-DABCYL; SEQ ID NO: 3), supplied at 100 µg/mL in YPD supplemented with 50 mM Tris, pH 7.0, 150 mM NaCl, and 1 mM EDTA. The secreted enzyme successfully cleaved the substrate, resulting in a fluorescent signal. The arrows in the left panel of Figure 15 point to cells in wells which correspond to the bright fluorescent wells observed in the right panel of Figure 15.

## Claims

1. A method of performing solution-phase enzyme screening, comprising:
depositing a library of cells onto a microdevice,
wherein said microdevice contains wells that spatially separate said cells in solution,
wherein said cells are distributed one cell per well on average,
wherein a plurality of the cells secrete variants of at least one enzyme in said solution;
contacting said secreted enzyme variants in said solution with at least one optical signal substrate;
wherein said enzyme activity is evaluated by detecting changes over time in one or more optical signals generated by said one or more optical signal substrates in the library of cells,
wherein such changes indicate a desired activity of the variants of the enzyme.

2. The method of claim 1, wherein said optical signal is a fluorescence signal.

3. The method of claim 2, wherein said enzyme activity is monitored in real-time or near-real-time in said microdevice on the basis of changes in the intensities of said fluorescent signal.

4. The method of claim 1, wherein said enzyme is selected from the group consisting of a protease, an oxidoreductase, a transferase, a hydrolase, a lyase, an isomerase, and a ligase.

5. The method of claim 4, wherein the molecular weight of said enzyme is greater than about 600 Da and less than about 100,000 Da.

6. The method of claim 1, wherein said enzyme activity is evaluated based on multiple parameters selected from the group consisting of catalytic rate, specificity of reaction, kinetic efficiency, and substrate binding affinity.

7. The method of claim 6, wherein said parameters are evaluated in parallel.

8. The method of claim 1, wherein said cells are eukaryotic cells.

9. The method of claim 8, wherein said eukaryotic cells are yeast cells.

10. The method of claim 1, wherein said wells are between about 10 and about 100 µm in diameter.

11. The method of claim 1, further comprising generating said variants by a step comprising mutating the gene encoding the enzyme.

12. The method of claim 1, further comprising retrieving said cells that secrete a desired enzyme variant from said microdevice.

13. The method of claim 12, wherein said retrieving is by micromanipulation with a glass capillary.

14. The method of claim 13, further comprising isolating and sequencing the DNA encoding the desired enzyme variant.

15. The method of claim 2, wherein said enzyme is a mutant glycosyltransferase (GTase) or a glycosidase.

16. The method of claim 15, wherein said GTase is capable of competing with chemical synthesis for the rapid and large scale production of complex carbohydrates.

## Patentansprüche

1. Verfahren zur Durchführung eines Flüssigphasen-Enzym-Screenings, ausweisend:
das Ablegen einer Zellbibliothek auf einer Mikrovorrichtung,
wobei die genannte Mikrovorrichtung Mikrotiterplatten enthält, welche die Zellen, in der Lösung, räumlich trennen,
wobei die genannten Zellen eine durchschnittliche Verteilung von einer Zelle pro Mikrotiterplatte aufweisen,
wobei eine Mehrzahl der genannten Zellen Varianten mindestens eines Enzyms in die genannte Lösung sekretieren;
das in Kontakt bringen der genannten sekretierten Enzymvarianten in der genannten Lösung mit mindestens einem optischen Signalsubstrat;
wobei die genannte Enzymaktivität durch das Erfassen von Änderungen von einem oder mehreren optischen Signalen pro Zeiteinheit, erzeugt durch ein oder mehrere optische Signalsubstrate in der Zellbibliothek, bewertet wird,
wobei solche Änderungen eine gewünschte Aktivität der Enzymvarianten anzeigen.

2. Verfahren nach Anspruch 1, wobei das genannte optische Signal ein Fluoreszenzsignal ist.

3. Verfahren nach Anspruch 2, wobei die genannte Enzymaktivität in Echtzeit oder nahezu in Echtzeit in der genannten Mikrovorrichtung auf der Basis von Änderungen der Intensität von genanntem Fluoreszenzsignal überwacht wird.

4. Verfahren nach Anspruch 1, wobei das genannte Enzym aus der Gruppe bestehend aus einer Protease, einer Oxidoreduktase, einer Transferase, einer Hydrolase, einer Lyase, einer Isomerase und einer Ligase ausgewählt wird.

5. Verfahren nach Anspruch 4, wobei das Molekulargewicht des genannten Enzyms höher als ca. 600 Da und niedriger als ca. 100 000 Da ist.

6. Verfahren nach Anspruch 1, wobei die genannte Enzymaktivität auf Basis mehrerer Parameter, ausgewählt aus einer Gruppe bestehend aus katalytischer Rate, Spezifität der Reaktion, kinetischer Effizienz und Substratbindungsaffinität, bewertet wird.

7. Verfahren nach Anspruch 6, wobei die genannten Parameter parallel bewertet werden.

8. Verfahren nach Anspruch 1, wobei die genannten Zellen eukaryotische Zellen sind.

9. Verfahren nach Anspruch 8, wobei die genannten eukaryotischen Zellen Hefezellen sind.

10. Verfahren nach Anspruch 1, wobei die genannten Mikrotiterplatten zwischen ca. 10 und ca. 100 µm im Durchmesser sind.

11. Verfahren nach Anspruch 1, das ferner das Generieren der genannten Varianten in einem Schritt, welcher das Mutieren des für das Enzym kodierenden Gens beinhaltet, umfasst.

12. Verfahren nach Anspruch 1, das ferner die Wiedergewinnen, der genannten Zellen, welche eine gewünschte Enzymvariante sekretieren, aus der genannten Mikrovorrichtung umfasst.

13. Verfahren nach Anspruch 12, wobei die genannte Wiedergewinnung durch Mikromanipulation mit einer Glaskapillare erfolgt.

14. Verfahren nach Anspruch 13, das ferner das Isolieren und Sequenzieren der DNA, die für die gewünschte Enzymvariante codiert, umfasst.

15. Verfahren nach Anspruch 2, wobei das genannte Enzym eine Glycosyltransferasen (GTase) - Mutation oder eine Glycosidase ist.

16. Verfahren nach Anspruch 15, wobei die genannte GTase mit der chemischen Synthese für die rasche und großtechnische Herstellung von komplexen Kohlenhydraten konkurrieren kann.

## Revendications

1. Procédé de mise en oeuvre d'un criblage d'enzyme en phase de solution, comprenant :
le dépôt d'une banque de cellules sur un micro-dispositif,
où ledit micro-dispositif contient des puits qui séparent spatialement lesdites cellules en solution,
où lesdites cellules sont distribuées une cellule par puits en moyenne,
où une pluralité des cellules sécrètent des variantes d'au moins une enzyme dans ladite solution ;
la mise en contact desdites variantes d'enzyme sécrétées dans ladite solution avec au moins un substrat à signaux optiques ;
où ladite activité enzymatique est évaluée par détection de changements au cours du temps dans un ou plusieurs signaux optiques générés par lesdits un ou plusieurs substrats à signaux optiques dans la banque de cellules,
où de tels changements indiquent une activité souhaitée des variantes de l'enzyme.

2. Procédé selon la revendication 1, où ledit signal optique est un signal. de fluorescence.

3. Procédé selon la revendication 2, où ladite activité enzymatique est suivie en temps réel ou sensiblement en temps réel dans ledit micro-dispositif sur la base de changements dans les intensités dudit signal fluorescent.

4. Procédé selon la revendication 1, où ladite enzyme est choisie dans le groupe consistant en une protéase, une oxydoréductase, une transférase, une hydrolase, une lyase, une isomérase et une ligase.

5. Procédé selon la revendication 4, où la masse moléculaire de ladite enzyme est supérieure à environ 600 Da et inférieure à environ 100 000 Da.

6. Procédé selon la revendication 1, où ladite activité enzymatique est évaluée sur la base de paramètres multiples choisis dans le groupe consistant en la vitesse catalytique, la spécificité de réaction, l'efficacité cinétique et l'affinité de liaison au substrat.

7. Procédé selon la revendication 6, où lesdits paramètres sont évalués en parallèle.

8. Procédé selon la revendication 1, où lesdites cellules sont des cellules eucaryotes.

9. Procédé selon la revendication 8, où lesdites cellules eucaryotes sont des cellules de levure.

10. Procédé selon la revendication 1, où lesdites cellules ont un diamètre entre environ 10 et environ 100 µm.

11. Procédé selon la revendication 1, comprenant en outre la production desdites variantes par une étape comprenant la mutation du gène codant l'enzyme.

12. Procédé selon la revendication 1, comprenant en outre la récupération desdites cellules qui sécrètent une variante d'enzyme souhaitée à partir dudit micro-dispositif.

13. Procédé selon la revendication 12, où ladite récupération est par micromanipulation avec un capillaire en verre.

14. Procédé selon la revendication 13, comprenant en outre l'isolement et le séquençage de l'ADN codant la variante d'enzyme souhaitée.

15. Procédé selon la revendication 2, où ladite enzyme est une glycosyltransférase (GTase) mutante ou une glycosidase.

16. Procédé selon la revendication 15, où ladite GTase est capable d'entrer en compétition avec la synthèse chimique pour la production rapide et à grande échelle de glucides complexes.
